(19) Europäisches Patentamt
European Patent Office
Office européen des brevets

(11) Veröffentlichungsnummer: **0 201 452**
**B1**

(12) # EUROPÄISCHE PATENTSCHRIFT

(45) Veröffentlichungstag der Patentschrift: **09.08.89**

(51) Int. Cl.⁴: **C 07 J 1/00**

(21) Anmeldenummer: **86730074.1**

(22) Anmeldetag: **07.05.86**

(54) **Verfahren zur Herstellung von 17alpha-Ethinyl-17beta-hydroxy-18-methyl-4,15-estradien-3-on und die neuen Ausgangsverbindungen für dieses Verfahren.**

(30) Priorität: **10.05.85 DE 3517466**

(43) Veröffentlichungstag der Anmeldung: **12.11.86 Patentblatt 86/46**

(45) Bekanntmachung des Hinweises auf die Patenterteilung: **09.08.89 Patentblatt 89/32**

(84) Benannte Vertragsstaaten: **AT BE CH DE FR GB IT LI LU NL SE**

(56) Entgegenhaltungen: **FR–A– 2 326 927**

(73) Patentinhaber: **SCHERING AKTIENGESELLSCHAFT Berlin und Bergkamen Müllerstrasse 170/178 Postfach 65 03 11 D-1000 Berlin 65 (DE)**

(72) Erfinder: **Hofmeister, Helmut, Dr. Weislingenstrasse 4 D-1000 Berlin 28 (DE)**
Erfinder: **Laurent, Henry, Dr. Glambecker Weg 21 D-1000 Berlin 28 (DE)**
Erfinder: **Lorenz, Hans-Peter, Dr. Residenzstrasse 17/18 D-1000 Berlin 51 (DE)**
Erfinder: **Wiechert, Rudolf, Prof. Dr. Petzower Strasse 8A D-1000 Berlin 39 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 201 452 B1

Jouve, 18, rue St-Denis, 75001 Paris, France

**Beschreibung**

Die Erfindung betrifft ein neues Verfahren zur Herstellung von 17α-Ethinyl-17β-hydroxy-18-methyl-4,15-estradien-3-on (Gestoden) und die der neuen Ausgangsverbindungen für dieses Verfahren. Gestoden ist ein stark wirksames bekanntes Gestagen, das zum Beispiel in Präparaten zur Kontrazeption verwendet werden kann.

Es sind bereits mehrere Verfahren zur Herstellung von Gestoden bekannt. Die Herstellung erfolgt jeweils durch Ethinylierung eines 17-Ketons.

In der deutschen Patentschrift 2 546 062 werden Ethinylierungen an 17-Ketonen folgender allgemeiner Formel III beschrieben :

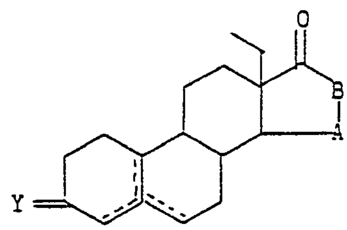

(III)

worin

Y eine freie oder vorzugsweise als Ketal geschützte Ketogruppe und eine der ⩶ Bindungen in 4,5-, 5,6- oder 5,10-Position eine Kohlenstoff-Kohlenstoff-Doppelbindung und die anderen je eine Kohlenstoff-Kohlenstoff-Einfachbindung und A-B eine Kohlenstoff-Kohlenstoff-Doppelbindung oder die Gruppierung

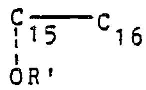

bedeuten, wobei R' ein Wasserstoffatom, eine Silyl-, Acyl-, Sulfonyl- oder Nitrogruppe darstellt.

Läßt man auf 18-Methyl-4,15-estradien-3,17-dion (Formel III : Y = O, A—B = CH=CH) Lithiumacetylid einwirken, so erhält man Gestoden in hoher Ausbeute von etwa 75 %. Diese Methode ist aber nur bei Substanzmengen bis maximal 100 g anwendbar; die Ethinylierung versagt, wenn sie auf diesem Wege in technischem Maßstab durchgeführt werden soll. Darüber hinaus wurde festgestellt, daß 18-Methyl-4,15-estradien-3,17-dion bei Hautkontakt sensibilisierend sein kann, so daß beim Umgang mit 18-Methyl-4,15-estradien-3,17-dion Schutzmaßnahmen beachtet werden müssen.

Die Gestoden-Herstellung über die 3-Ketale (Formel III : Y = Ethylendioxy oder 2,2-Dimethyl-1,3-propylendioxy) hat den Nachteil, daß die 3-Ketale als Isomerengemische in öliger Form anfallen, die sich nur auf chromatographischem Wege reinigen lassen.

In der deutschen Patentschrift 2 749 104 wird die Ethinylierung an 17-Ketonen beschrieben, bei denen die 3-Ketogruppe mit Ethandithiol-(1,2) geschützt ist (Formel III : Y = Ethylendithio mit einer Kohlenstoff-Kohlenstoff-Doppelbindung in 4,5-Position). Ethandithiol stellt schon in kleinen Mengen wegen des unangenehmen Geruchs eine Umweltbelastung dar. Weiterhin hat die Thioketal-Schutzgruppe den Nachteil, daß sie sich nach der Ethinylierung nur mit einem großen Überschuß an Methyljodid, einem teuren Reagenz, abspalten läßt. Damit werden die Vorteile der Thioketale, die in der leichten Kristallisierbarkeit und fast quantitativen Umsetzbarkeit liegen, wieder aufgehoben.

Die Nachteile der bekannten Verfahren lassen es notwendig erscheinen, nach einem besseren Herstellungsverfahren für Gestoden zu suchen.

Es wurde nun gefunden, daß die neuen 17-Ketone der allgemeinen Formel II

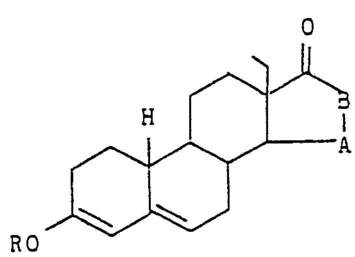

(II)

worin

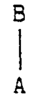 für die Gruppierung

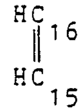

oder

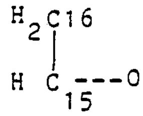

mit R′ in der Bedeutung einer bis zu 10 Kohlenstoffatome enthaltenden Acylgruppe
stehen,
gut kristallisierende Verbindungen darstellen, die für die Herstellung von Gestoden geeignet sind, wenn R für einen Alkylrest mit 1 bis 3 Kohlenstoffatomen steht.

Die Erfindung betrifft somit ein neues Verfahren zur Herstellung von 17α-Ethinyl-17β-hydroxy-18-methyl-4,15-estradien-3-on (Gestoden) durch Ethinylierung von Verbindungen der allgemeinen Formel II sowie die Verbindungen der allgemeinen Formel II als neue Ausgangsprodukte der Gestodensynthese.

In Formel II steht R für einen Alkylrest mit 1 bis 3 Kohlenstoffatomen, wobei der Methyl- und Ethylrest bevorzugt sind. Als Acylreste R′ kommen die Reste organischer Carbonsäuren mit bis zu 10 Kohlenstoffatomen infrage; beispielsweise genannt seien der Acetyl-, Trifluoracetyl-, Trimethylacetyl-, Propionyl-, Butyryl-, Heptanoyl- und Benzoylrest, wobei der Acetylrest bevorzugt ist.

Die Ethinylierung des 17-Ketons der allgemeinen Formel II erfolgt mit Lithiumacetylid in an sich bekannter Weise. Lithiumacetylid kann auch in situ gebildet und mit dem 17-Keton der Formel II zur Reaktion gebracht werden. So kann man zum Beispiel auf das 17-Keton in einem geeigneten Lösungsmittel wie Tetrahydrofuran eine Lösung von Alkyllithium und Acetylen in Tetrahydrofuran und Hexan oder Tetrahydrofuran und Diethylether bei Temperaturen zwischen — 70 °C und Raumtemperatur einwirken lassen. Alkyllithium wird vorzugsweise als n-Butyllithium in 15 %iger Lösung von Hexan oder als Methyllithium in etherischer Lösung eingesetzt. Eine im 17-Keton enthaltene Gruppe OR′ wird unter der Ethinylierung als HOR′ zur 15,16-Doppelbindung abgespalten. Die anschließende Spaltung der 3-Dienolethergruppe zur 3-Ketogruppe wird nach den dem Fachmann bekannten Methoden durchgeführt. Zur Dienoletherspaltung kommen beispielsweise Mineralsäuren, wie zum Beispiel Perchlorsäure, Schwefelsäure oder Salzsäure, oder organische Säuren, wie zum Beispiel Oxalsäure, in Betracht. Die Spaltung wird vorzugsweise in alkoholischer Lösung bei Temperaturen zwischen etwa 20 und 100 °C durchgeführt.

Die neuen 17-Ketone der allgemeinen Formel II werden aus dem in der deutschen Patentschrift 2 546 062 beschriebenen 15α-Hydroxy-18-methyl-4-estren-3,17-dion entweder durch Wasserabspaltung aus 15,16-Stellung und Überführung in den 3-Dienolether oder durch Acylierung der 15α-Hydroxygruppe und Überführung in den 3-Dienolether hergestellt. Die Wasserabspaltung zur 15,16-Doppelbindung erfolgt in an sich bekannter Weise mit Methansulfonylchlorid in Pyridin über das 15α-Mesylat und weitere Behandlung des Mesylats mit Dimethylformamid und wasserfreiem Natriumacetat.

Die selektive Überführung des 3,17-Diketons in den 3-Dienolether (R = Alkyl mit 1-3 Kohlenstoffatomen) gelingt mit Trialkylorthoformiat in Dioxan oder mit 2,2-Dialkoxypropan in Dimethylformamid, Tetrahydrofuran oder Dioxan in Gegenwart einer Säure wie p-Toluolsulfonsäure oder Pyridiniumtosylat.

Für die Acylierung der 15α-Hydroxygruppe, die in üblicher Weise abläuft, sei beispielsweise die Umsetzung mit einem entsprechenden Carbonsäureanhydrid oder -chlorid (R′ = Acylgruppe mit bis zu 10 Kohlenstoffatomen) in Gegenwart eines tertiären Amins genannt. Als tertiäre Amine sind Pyridin und Dimethylaminopyridin bzw. Gemische aus Pyridin und Dimethylaminopyridin besonders geeignet.

Die Herstellung der neuen 17-Ketone der allgemeinen Formel II wird anhand der folgenden Beispiele näher erläutert.

Vorschrift I

a) 18-Methyl-4,15-estradien-3,17-dion

Zu 250 g 15α-Hydroxy-18-methyl-4-erstren-3,17-dion in 704 ml Pyridin werden bei 0 °C 77,5 ml Methansulfonylchlorid innerhalb 10 Minuten getropft. Nach 3,5 Std. gibt man 350 ml Dimethylformamid und 283 g wasserfreies Natriumacetat zu, läßt unter Argon 20 Std. bei Raumtemperatur rühren und gibt das Reaktionsgemisch in Eiswasser. Das ausgefallene Produkt wird abgesaugt, mit Wasser gewaschen und im Vakuum getrocknet. Zur Reinigung behandelt man das Rohprodukt (212 g) in 1 500 ml Essigester und 0,4 ml Pyridin mit 21 g Aktiv-Kohle. Nach dem Einengen der Lösung auf 420 ml kristallisieren bei 0 °C 188 g 18-Methyl-4,15-estradien-3,17-dion aus. Schmelzpunkt 156,9 °C (aus Aceton/Hexan).

b) 3-Methoxy-18-methyl-3,5,15-estratrien-17-on

20,0 g 18-Methyl-4,15-estradien-3,17-dion in 100 ml 1,4-Dioxan und 100 ml Trimethylorthoformiat werden unter Argon mit 800 mg p-Toluolsulfonsäure bei 50 °C gerührt. Nach 2,5 Std. gibt man 10 ml Pyridin zu und destilliert die Lösung im Vakuum weitgehend ein. Der Rückstand wird in Essigester gelöst, mit Wasser gewaschen und getrocknet. Nach Chromatographieren des Rohproduktes mit 0-20 % Essigester-Hexan erhält man 14,0 g 3-Methoxy-18-methyl-3,5,15-estratrien-17-on vom Schmelzpunkt 162,1 °C (aus Methanol).

Vorschrift II

a) 15α-Acetoxy-18-methyl-4-estren-3,17-dion

Zu 100 g 15α-Hydroxy-18-methyl-4-estren-3,17-dion in 350 ml Pyridin tropft man 200 ml Essigsäureanhydrid. Nach 1 Std. wird das Reaktionsgemisch in Eiswasser eingerührt. Das ausgefallene Produkt wird abgesaugt, mit Wasser neutral gewaschen und im Vakuum bei 50 °C getrocknet. Es werden 100 g 15α-Acetoxy-18-methyl-4-estren-3,17-dion erhalten. Eine aus Aceton/Hexan umkristallisierte Probe schmilzt bei 165 °C.

b) 15α-Acetoxy-3-methoxy-18-methyl-3,5-estradien-17-on

60,0 g 15α-Acetoxy-18-methyl-4-estren-3,17-dion in 500 ml DMF werden mit 500 ml 2,2-Dimethoxypropan, 20 ml Methanol und 6,0 g Pyridiniumtosylat bei 110 °C unter Argon gerührt. Nach 3,5 Std. gibt man 9,0 g festes Natriumhydrogencarbonat zu, rührt das Reaktionsgemisch in 10 Liter Eis/Wasser ein, saugt das ausgefallene Produkt ab, wäscht mehrmals mit Wasser und trocknet im Vakuum bei 70 °C. Das Rohprodukt wird in 375 ml Methanol suspendiert und unter Rückfluß 15 Minuten gerührt. Nach dem langsamen Abkühlen auf Raumtemperatur saugt man das farblose Kristallisat ab und wäscht mit kaltem Methanol nach. Ausbeute : 56 g 15α-Acetoxy-3-methoxy-18-methyl-3,5-estradien-17-on. Schmelzpunkt 190 °C.

Vorschrift III

15α-Acetoxy-3-ethoxy-18-methyl-3,5-estradien-17-on

2,0 g 15α-Acetoxy-18-methyl-4-erstren-3,17-dion in 60 ml Dioxan werden mit 6 ml o-Ameisensäuretriethylester und 40 mg p-Toluolsulfonsäure 20 Std. bei Raumtemperatur unter Argon gerührt. Nach Zugabe von 2 ml Pyridin verdünnt man mit Ether, wäscht mit Wasser und trocknet. Das Rohprodukt wird aus Aceton/Hexan umkristallisiert.

Ausbeute : 1,6 g 15α-Acetoxy-3-ethoxy-18-methyl-3,5-estradien-17-on.

Schmelzpunkt : 202 °C.

Vorschrift IV

a) 15α-Benzoyloxy-18-methyl-4-estren-3,17 dion

Zu 5 g 15α-Hydroxy-18-methyl-4-estren-3,17-dion in 20 ml Pyridin gibt man bei 0 °C tropfenweise 5 ml Benzoylchlorid. Nach 45 Minuten setzt man 2 ml Wasser zu, rührt weitere 30 Minuten und gibt das Reaktionsgemisch dann in Eis/Wasser. Das ausgefallene Produkt wird abgesaugt, in Methylenchlorid gelöst, mit Wasser gewaschen und über Natriumsulfat getrocknet. Nach Chromatographieren des Rohproduktes an Kieselgel mit einem Aceton-Hexan-Gradienten werden 5,6 g 15α-Benzoyloxy-18-methyl-4-estren-3,17-dion als Schaum erhalten.

b) 15α-Benzoyloxy-3-methoxy-18-methyl-3,5-estradien-17-on

7,8 g 15α-Benzoyloxy-18-methyl-4-estren-3,17-dion werden in Analogie zu Vorschrift II b) mit Dimethoxypropan umgesetzt und aufgearbeitet. Es werden 6,8 g 15α-Benzoyloxy-3-methoxy-18-methyl-3,5-estradien-17-on als Schaum erhalten.

Vorschrift V

a) 18-Methyl-15α-trimethylacetoxy-4-estren-3,17-dion

10 g 15α-Hydroxy-18-methyl-4-estren-3,17-dion in 200 ml Pyridin werden mit 2 g Dimethylaminopyridin und 20 ml Pivalinsäureanhydrid versetzt und 1 Std. bei 50 °C gerührt. Man gibt das Gemisch in Eis/Wasser, extrahiert mit Essigester, wäscht mit Wasser neutral und trocknet über Natriumsulfat. Nach

Chromatographieren des Rohproduktes an Kieselgel mit einem Aceton-Hexan-Gradienten werden 9,5 g 18-Methyl-15α-trimethylacetoxy-4-estren-3,17-dion als Schaum erhalten.

b) 3-Methoxy-18-methyl-15α-trimethylacetoxy-3,5-estradien-17-on

5,6 g 18-Methyl-15α-trimethylacetoxy-4-estren-3,17-dion werden in Analogie zu Vorschrift II b) mit Dimethoxypropan umgesetzt und aufgearbeitet. Man erhält 4,9 g 3-Methoxy-18-methyl-15α-trimethylacetoxy-3,5-estradien-17-on.

Beispiel 1

17α-Ethinyl-17β-hydroxy-18-methyl-4,15-estradien-3-on

a) Aus 3-Methoxy-18-methyl-3,5,15-estratrien-17-on

In eine Lösung von 40 ml n-Butyllithium (15proz. in Hexan) in 100 ml abs. Tetrahydrofuran leitet man bei 0 °C 15 Minuten Acetylen ein und tropft dann 4,0 g 3-Methoxy-18-methyl-3,5,15-estratrien-17-on in 40 ml Tetrahydrofuran zu. Nach 15 Minuten versetzt man mit 8 ml Wasser, 40 ml Methanol und 8 g Oxalsäure, rührt 1 Std. bei 60 °C, gibt 600 ml Wasser zu und extrahiert mit Essigester. Die organische Phase wird mit Wasser gewaschen und getrocknet. Es werden nach Umkristallisieren aus Essigester 2,4 g 17α-Ethinyl-17β-hydroxy-18-methyl-4,15-estradien-3-on erhalten. Schmelzpunkt 197 °C.

b) Aus 15α-Acetoxy-3-methoxy-18-methyl-3,5-estradien-17-on

In eine Lösung von 50 ml n-Butyllithium (15proz. in Hexan) in 130 ml abs. Tetrahydrofuran leitet man 15 Minuten Acetylen ein. Die so erhaltene Lithiumacetylid-Lösung tropft man bei 0 °C unter Argon zu 5,0 g 15α-Acetoxy-3-methoxy-18-methyl-3,5-estradien-17-on in 50 ml Tetrahydrofuran. Nach vollständiger Zugabe der Lithiumacetylid-Lösung rührt man 15 Minuten, versetzt dann vorsichtig mit 20 ml 50proz. Salzsäure, rührt weitere 30 Minuten und verdünnt anschließend das Reaktionsgemisch mit Essigester. Die organische Phase wird mehrmals mit Wasser gewaschen, über Natriumsulfat getrocknet und im Vakuum eingeengt. Das Rohprodukt behandelt man in Aceton mit Aktiv-Kohle und kristallisiert aus Aceton/Hexan um. Ausbeute 3,7 g 17α-Ethinyl-17β-hydroxy-18-methyl-4,15-estradien-3-on vom Schmelzpunkt 197,8 °C.

c) Aus 15α-Benzoyloxy-3-methoxy-18-methyl-3,5-estradien-17-on

3,6 g 15α-Benzoyloxy-3-methoxy-18-methyl-3,5-estradien-17-on werden wie unter 1b) beschrieben mit Lithiumacetylid umgesetzt. Nach erfolgter Reaktion versetzt man das Reaktionsgemisch mit gesättigter Ammoniumchlorid-Lösung, verdünnt mit Essigester, wäscht mit Wasser und trocknet über Natriumsulfat. Das erhaltene 17α-Ethinyl-3-methoxy-18-methyl-3,5,15-estratrien-17β-ol wird als Rohprodukt in 70 ml Methanol und 6 ml Wasser mit 3 g Oxalsäure 10 Minuten unter Rückfluß gerührt. Das Reaktionsgemisch wird in Eis/Wasser gegeben. Das ausgefallene Produkt wird abgesaugt, in Essigester gelöst, mit Wasser gewaschen und über Natriumsulfat getrocknet. Nach Umkristallisieren aus Aceton/Hexan werden 1,9 g 17α-Ethinyl-17β-hydroxy-18-methyl-4,15-estradien-3-on vom Schmelzpunkt 197,8 °C erhalten.

d) Aus 3-Methoxy-18-methyl-15α-trimethylacetoxy-3,5-estradien-17-on

2,8 g 3-Methoxy-18-methyl-15α-trimethylacetoxy-3,5-estradien-17-on werden analog Beispiel 1b) mit Lithiumacetylid umgesetzt und aufgearbeitet. Man erhält nach Umkristallisieren aus Aceton/Hexan 1,6 g 17α-Ethinyl-17β-hydroxy-18-methyl-4,15-estradien-3-on vom Schmelzpunkt 197 °C.

**Patentansprüche** (für die Vertragsstaaten : BE, CH, DE, FR, GB, IT, LI, LU, NL, SE)

1. Verfahren zur Herstellung von 17α-Ethinyl-17β-hydroxy-18-methyl-4,15-estradien-3-on der Formel I

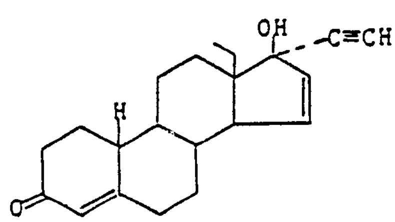

(I)

bei dem ein 17-Keton der allgemeinen Formel II

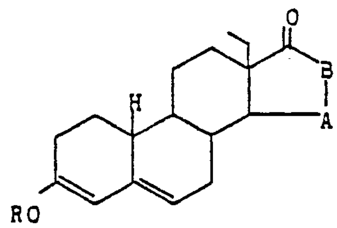

(II)

worin

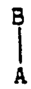 die Gruppierung

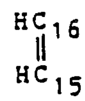

oder

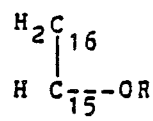

bedeuten,
wobei R' eine Acylgruppe mit bis zu 10 Kohlenstoffatomen darstellt,
mit Lithiumacetylid ethinyliert, wobei HOR' zur 15,16-Doppelbindung abgespalten wird, und anschließend durch Einwirkung einer Säure den 3-Dienolether zum 3-Keton gespalten wird, dadurch gekennzeichnet, daß ein 17-Keton der allgemeinen Formel II verwendet wird, worin R für einen Alkylrest mit 1 bis 3 Kohlenstoffatomen steht.

2. Verbindungen der allgemeinen Formel II

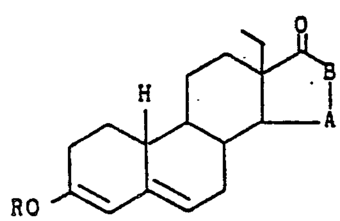

(II)

worin
einen Alkylrest mit 1-3 Kohlenstoffatomen und

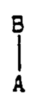 die Gruppierung

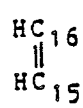

oder

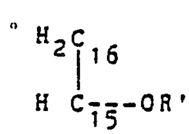

bedeuten,
wobei R' eine Acylgruppe mit bis zu 10 Kohlenstoffatomen darstellt.

3. 3-Methoxy-18-methyl-3,5,15-estratrien-17-on.
4. 15α-Acetoxy-3-methoxy-18-methyl-3,5-estradien-17-on.
5. 15α-Acetoxy-3-ethoxy-18-methyl-3,5-estradien-17-on.
6. 15α-Benzoyloxy-3-methoxy-18-methyl-3,5-estradien-17-on.
7. 3-Methoxy-18-methyl-15α-trimethylacetoxy-3,5-estradien-17-on.

**Patentanspruch** (für den Vertragsstaat AT)

Verfahren zur Herstellung von 17α-Ethinyl-17β-hydroxy-18-methyl-4,15-estradien-3-on der Formel I

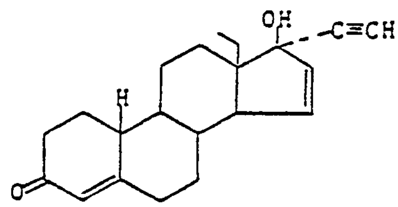

(I)

bei dem ein 17-Keton der allgemeinen Formel II

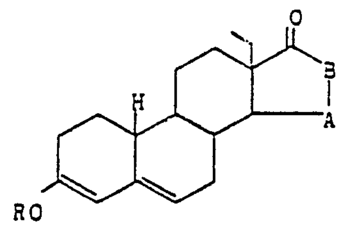

(II)

worin

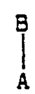 die Gruppierung

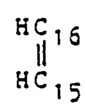

oder

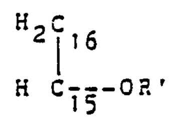

bedeuten,
wobei R′ eine Acylgruppe mit bis zu 10 Kohlenstoffatomen
darstellt,
mit Lithiumacetylid ethinyliert, wobei HOR′ zur 15,16-Doppelbindung abgespalten wird, und anschließend durch Einwirkung einer Säure den 3-Dienolether zum 3-Keton gespalten wird, dadurch gekennzeichnet, daß ein 17-Keton der allgemeinen Formel II verwendet wird, worin R für einen Alkylrest mit 1 bis 3 Kohlenstoffatomen steht.

**Claims** (for the Contracting States : BE, CH, DE, FR, GB, IT, LI, LU, NL, SE)

1. Process for the manufacture of 17α-ethynyl-17β-hydroxy-18-methyl-4,15-œstradien-3-one of the formula I

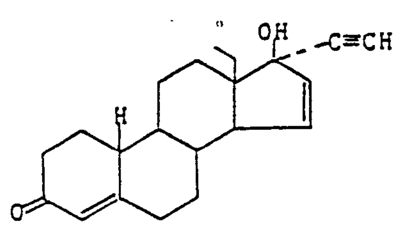

(I)

wherein a 17-ketone of the general formula II

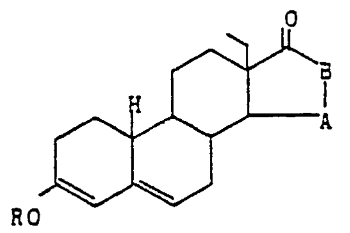

(II)

in which

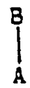 represents the grouping

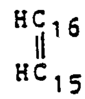

or

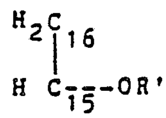

in which R′ represents an acyl group containing up to 10 carbon atoms, is ethynylated with lithium acetylide, HOR′ being split off to form the 15,16-double bond, and then, by the action of an acid, the 3-dienol ether is cleaved to the 3-ketone, characterised in that a 17-ketone of the general formula II is used in which R represents an alkyl radical having from 1 to 3 carbon atoms.

2. Compounds of the general formula II

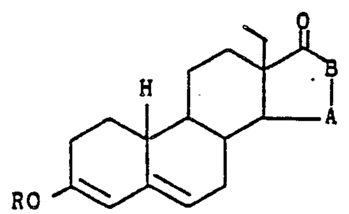

(II)

in which

R represents an alkyl radical having from 1 to 3 carbon atoms

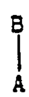 represents the grouping

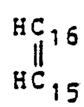

or

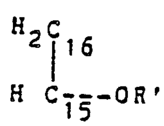

in which R′ represents an acyl group containing up to 10 carbon atoms.

3. 3-Methoxy-18-methyl-3,5,15-œstratrien-17-one.
4. 15α-Acetoxy-3-methoxy-18-methyl-3,5-œstradien-17-one.
5. 15α-Acetoxy-3-ethoxy-18-methyl-3,5-œstradien-17-one.
6. 15α-Benzoyloxy-3-methoxy-18-methyl-3,5-œstradien-17-one.
7. 3-Methoxy-18-methyl-15α-trimethylacetoxy-3,5-œstradien-17-one.

**Claim** (for the Contracting State AT)

Process for the manufacture of 17α-ethynyl-17β-hydroxy-18-methyl-4,15-œstradien-3-one of the formula I

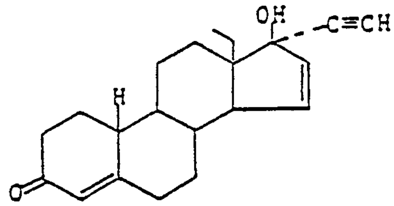

(I)

wherein a 17-ketone of the general formula II

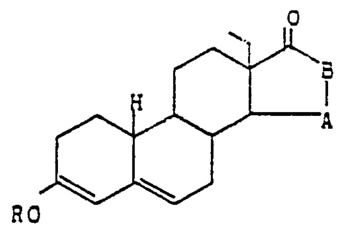

(II)

in which

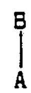 represents the grouping

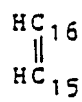

or

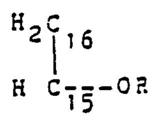

in which R′ represents an acyl group containing up to 10 carbon atoms, is ethynylated with lithium acetylide, HOR′ being split off to form the 15,16-double bond, and then, by the action of an acid, the 3-dienol ether is cleaved to the 3-ketone, characterised in that a 17-ketone of the general formula II is used in which R represents an alkyl radical having from 1 to 3 carbon atoms.

**Revendications** (pour les Etats contractants : BE, CH, DE, FR, GB, IT, LI, LU, NL, SE)

1. Procédé de préparation de 17α-éthynyl-17β-hydroxy-18-méthyl-4,15-œstradién-3-one répondant à la formule I

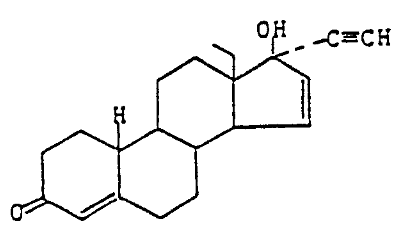

(I)

dans lequel on éthynyle avec de l'acétylure de lithium une 17-cétone de la formule générale II

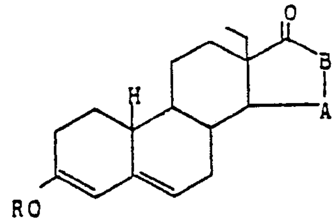

(II)

dans laquelle

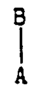 représente le groupement

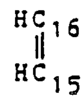

ou

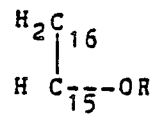

où R' représente un groupe acyle comportant jusqu'à 10 atomes de carbone, HOR' étant séparé pour former une double liaison 15,16, et on sépare ensuite le 3-diénoléther pour former la 3-cétone par l'action d'un acide, caractérisé en ce qu'on utilise une 17-cétone de la formule générale II dans laquelle R représente un radical alkyle comportant 1 à 3 atomes de carbone.

2. Composés de la formule générale II

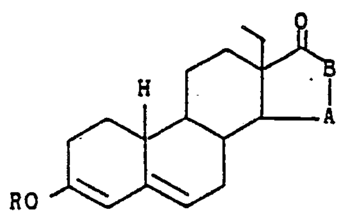

(II)

dans laquelle R représente un radical alkyle comportant 1 à 3 atomes de carbone et

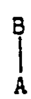 représente le groupement

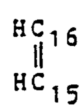

ou

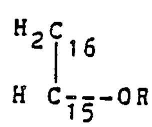

R' représentant un groupe acyle comportant jusqu'à 10 atomes de carbone.

3. La 3-méthoxy-18-méthyl-3,5,15-œstratrién-17-one.
4. La 15α-acétoxy-3-méthoxy-18-méthyl-3,5-œstradién-17-one.
5. La 15α-acétoxy-3-éthoxy-18-méthyl-3,5-œstradién-17-one.
6. La 15α-benzoyloxy-3-méthoxy-18-méthyl-3,5-œstradién-17-one.
7. La 3-méthoxy-18-méthyl-15α-triméthylacétoxy-3,5-œstradién-17-one.

**Revendication** (pour l'Etat contractant AT)

Procédé de préparation de 17α-éthynyl-17β-hydroxy-18-méthyl-4,15-œstradién-3-one répondant à la formule I

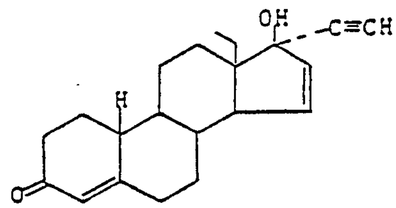

dans lequel on éthynyle avec de l'acétylure de lithium une 17-cétone de la formule générale II

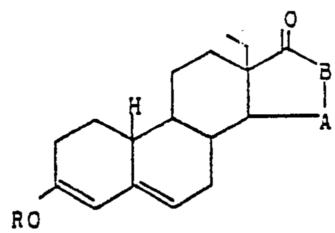

dans laquelle

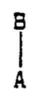 représente le groupement

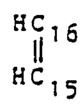

ou

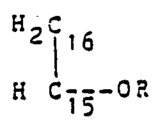

où R' représente un groupe acyle comportant jusqu'à 10 atomes de carbone, HOR' étant séparé pour former une double liaison 15, 16, et on sépare ensuite le 3-diénoléther pour former la 3-cétone par l'action d'un acide, caractérisé en ce qu'on utilise une 17-cétone de la formule générale II dans laquelle R représente un radical alkyle comportant 1 à 3 atomes de carbone.